# EUROPEAN PATENT APPLICATION

(11) **EP 4 623 969 A1**
(43) Date of publication of application: **01.10.2025**
(21) Application number: 24167863.0
(22) Date of filing: 29.03.2024
(51) Int. Cl.: A61M 11/06

(54) **NEBULIZER WITH A DETACHABLE FILTER MODULE USEABLE IN AEROSOLTHERAPY**

(71) Applicant: Air Liquide Medical Systems S.r.l., 20152 Milano (IT)
(72) Inventor: SIGNORINI, Alice, 25073 Bovezzo (IT); RUOCCO, Alessandra, 25073 Bovezzo (IT); ALBERICI, Luca, 25073 Bovezzo (IT); SANDONI, Giuseppe, 25073 Bovezzo (IT)
(74) Representative: Air Liquide

(57) **Abstract**

The invention concerns a nebulizer (1) for aerosolizing a liquid comprising a base part (10) comprising a tubular body (15) comprising a liquid chamber (11) for storing a liquid to be aerosolized and a central tubular structure (14) projecting upwardly in the liquid chamber (11), and a top part (20) comprising an elongated tubular portion (23) comprising an inner channel (24), and an aerosol-generating system (21) comprising a jet nozzle (26). The top part (20) is detachably secured to the base part (10). A filter module (40) is detachably attached to the top part (20). The filter module (40) comprises a module body (41) comprising an inner chamber (46) with a filter material (47) and the inner chamber (46) of the module body (41) is in fluid communication with the inner channel (24) of the elongated tubular portion (23).

## Description

The present invention concerns a nebulizer designed for being hold in hand by a user, such as a patient in need of an aerosoltherapy treatment, useable in aerosoltherapy for aerosolizing or nebulizing a liquid, such as a drug- or medication-containing liquid or solution, and an aerosoltherapy installation comprising such a nebulizer driven by an air-compressing device.

Nebulizing devices, commonly called nebulizers, are well-known devices useable for delivering an inhalation therapy, i.e. an aerosoltherapy, to a person, typically a patient, in need thereof. Nebulizers are used in combination with a source of compressed gas, typically compressed air, for nebulizing a liquid solution, typically a liquid-drug solution, thereby obtaining a mist or aerosol containing droplets having suitable sizes allowing them reaching the lower airways of the patient that has to inhale said mist in the course of his/her aerosoltherapy. The source of compressed gas is commonly an air-compressing device comprising a motorized compressing system that sucks, compresses ambient air and provides the thus obtained compressed-air flow to the nebulizer.

Usually, a nebulizer comprises an inner liquid chamber, commonly called an ampoule chamber, having an inner volume of several millimeters (mL), for instance of les than 20 mL, for receiving and/or storing the liquid or solution to be nebulized, and further an aerosol-generating system for generating an aerosol or mist. The aerosol-generating system typically comprises a jet nozzle having a delivery or jet orifice for providing a jet of gas/liquid jet formed by compressed air provided by the air-compressing device and some liquid sucked out of the ampoule chamber. The gas/liquid jet passing through the delivery orifice of the jet nozzle is delivered toward an impactor, also called a pisper, a deflector or the like, and the aerosol or mist is created upon impact of the gas/liquid jet on said impactor.

Examples of such nebulizers are given by EP3912662, EP3459577, EP3721928 and DE199002847, whereas an example of compressing device driven by an electric motor useable for providing compressed air to a nebulizer is given by EP4206467.

Some nebulizers are made of several parts or sub-units, such as a top part and a base part with the liquid chamber, that are detachably coupled together, for instance plugged, screwed, nested or the like, as they have to be frequently cleaned, for instance every day, or refilled with liquid-drug solution or the like.

During inspiration phases, the user, i.e. a patient or the like, inhales the aerosol or mist that contains medication/drug compounds carried by an airflow, while during expiration phases, the user exhales CO₂-enriched gases, i.e. exhaled gases containing CO₂ and water vapor. Besides CO₂ and water vapor, exhaled gases still contain some medication/drug compounds that have not being "consumed" in the patient's airways, i.e. not deposited, not used or the like. They are usually vented to the atmosphere.

A problem is that the medication/drug compounds that are vented to the atmosphere tend to accumulate in the room and may be re-inhaled by the user him/self, their relatives or other persons that do not need to be treated by those compounds.

A goal of the present invention is to solve or at least to reduce the above problem in providing an improved nebulizer that allows limiting the proportion of medication/drug compounds that are vented to the atmosphere.

A solution according to the present invention concerns a nebulizer, i.e. a nebulizing device, for aerosolizing, i.e. nebulizing, a liquid, typically a drug-containing solution, comprising :
- a base part comprising a tubular body comprising a liquid chamber for storing the liquid to be aerosolized, typically a drug-containing solution, and a tubular structure projecting upwardly in the liquid chamber, and
- a top part comprising an elongated tubular portion comprising an inner channel, and an aerosol-generating system, said aerosol-generating system comprising a jet nozzle arranged as a sleeve around at least a part of the tubular structure of the base part, and
wherein the top part is detachably secured to the base part.

Further, the nebulizer of the present invention further comprises a filter module detachably attached to the top part, wherein:
- the filter module comprises a module body comprising an inner chamber (i.e. a hollow compartment) comprising a (or several) filter material and
- the inner chamber of the module body is in fluid communication with the inner channel of the elongated tubular portion.

In relation to the present invention :
- an "aerosol" is a mist containing droplets of liquid, for instance a liquid drug, and a gas, such as air,
- a "gas" is formed of a unique compound (i.e. a pure gas) or of several compounds (i.e. gas mixture) that is/are in gaseous form.
- a "liquid drug" or a "drug-containing solution" is a solution that comprises one or several compound(s) and/or medication(s), such as one or several solid compounds or medications mixed, dissolved, suspended or similar in a liquid.
- the following terms are considered as being strictly equivalent:
   - "aerosol" and "mist",
   - "to aerosolize", "to atomize" and "to nebulize",
   - "compressed air" and "pressurized air",
   - "ampoule chamber", "liquid chamber" and "solution chamber",
   - "drug" and "medication",
   - "liquid" and "solution",
   - "particles" and "droplets",
   - "nebulizer", "nebulizing device" and "nebulization device",
   - "impactor", "pisper" and "deflector".

Depending on the embodiment, the nebulizer according to the present invention can comprise one or several of the following additional features:
- the inner channel comprises an upper outlet arranged in a tubular top end of the elongated tubular portion.
- the module body of the filter module comprises a first and a second opening in fluid communication with the inner chamber.
- the first opening is arranged on a first tubular connector.
- the second opening is arranged on a second tubular connector.
- the first and second tubular connectors are integral with, e.g. firmly attached or secured to, the module body of the filter module.
- the first and second tubular connectors can have a same or a different structure, in particular equal or non-equal diameter.
- when the filter module is attached to the top part, the first tubular connector of the module body is in fluid communication with the inner channel of the elongated tubular portion, and with the inner chamber of the module body.
- the first tubular connector of the module body is plugged onto the tubular top end of the elongated tubular portion, when the filter module is attached to the top part.
- the top part comprises an enlarged head comprising the tubular top end of the elongated tubular portion.
- the tubular top end is arranged at the center of the enlarged head.
- the filter material comprises polymer fibers.
- the filter material comprises polymer fibers of styrene butadiene copolymer (SBC) or polypropylene.
- the second opening of the second tubular connector is in fluid communication with the ambient atmosphere.
- the elongated tubular portion comprises a tube, a conduit or the like.
- the module body is made of a plastic material or the like, such as polycarbonate (PC), polypropylene (PP) or polyamide (PA).
- the module body has a cylindrical shape with a diameter D of between about 2 and 8 cm, and a thickness T of between about 2 and 10 cm.
- the filter module is axially-arranged on the top part, i.e. axis XX.
- the top part is "sandwiched" between the filter module and the base part of the nebulizer.

Depending on the embodiment, the nebulizer according to the present invention can further comprise one or several of the following additional features:
- the top part is axially-arranged (i.e. axis XX), i.e. inserted, in the base part.
- the top part further comprises at least one protruding element projecting radially and outwardly, said at least one protruding element being arranged in a top end region of the elongated tubular portion.
- the base part further comprises at least one abutment wall projecting radially and inwardly, said at least one abutment wall cooperating with said at least one protruding element for maintaining the top part secured to the base part, i.e. detachably attached thereto.
- the top part and the base part are arranged on a common axis XX, i.e. a vertical axis XX.
- the top part and the base part comprise (generally-) elongated shapes projecting along said vertical axis XX.
- the top part comprises at least two protruding elements, i.e. two or more protruding elements.
- preferably, the top part comprises a first and a second protruding element, namely two protruding elements.
- the base part comprises at least two abutment walls.
- preferably, the base part preferably a first and a second abutment wall, namely two abutment walls.
- the number of protruding elements equals the number of abutment walls.
- the first and second abutment walls (i.e. respectively) of the base part cooperate with the first and second protruding elements (i.e. respectively) of the top part for maintaining the top part secured to the base part, i.e. when the top part and the base part are coupled together.
- the first and second abutment walls are diametrically-opposite arranged in the tubular body of the base part, i.e. angularly spaced by an angle of about 180°.
- the first and a second protruding elements are diametrically-opposite arranged on the elongated tubular portion of the top part, i.e. angularly spaced by an angle of about 180°.
- the top part is mobile (at least) in rotation with respect to the base part between at least: a locking position, wherein the top part is secured, i.e. locked, to the base part, and an unlocking position, wherein the top part is released, i.e. unlocked, and can be detached, i.e. uncoupled/separated, from the base part.
- the top part comprises an enlarged head, preferably an enlarged head having a disk-shape or the like.
- said at least one protruding element are arranged on said enlarged head, preferably said first and second protruding elements.
- the enlarged head is arranged at the upper end of the top part, preferably at the top end region of the elongated tubular portion of the top part.
- the elongated tubular portion of the top part is topped by the enlarged head.
- the top part comprises, i.e. is axially (XX) traversed by, an inner channel comprising an upper outlet.
- the inner channel is arranged in the elongated tubular portion of the top part, i.e. the inner channel constitutes at least a part of the lumen of said elongated tubular portion.
- the upper outlet is arranged in said enlarged head, preferably the inner channel is axially-arranged (XX) in said top part.
- the upper outlet opens in the ambient atmosphere, i.e. it fluidly communicates with the ambient atmosphere.
- the inner channel fluidly communicates with the ambient atmosphere through said upper outlet.
- the enlarged head comprises an outer periphery (e.g. outer perimeter) comprising at least one anti-slipping region, such as one or several textured regions, for allowing users to manually-grab the enlarged head with the fingers of one of their hands and manipulate it, especially turn it clockwise or counterclockwise, without slipping. In other words, their fingers do not slip due to humidity, sweat or the like, thanks to the anti-slipping regions, e.g. textured region(s).
- the enlarged head comprises several anti-slipping regions, i.e. several textured regions, preferably two anti-slipping regions diametrically-opposed.
- the aerosol-generating system of the top part further comprises a deflector facing a jet outlet of the jet nozzle.
- the deflector is arranged in the inner channel of the top part.
- the deflector comprises a blade, a little wall or any other structure.
- the jet nozzle of the top part is arranged as a sleeve around the tubular structure of the base part, while maintaining a spacing in-between so that liquid stored in the liquid chamber can travel upwardly in said spacing toward the jet outlet of the jet nozzle.
- the spacing located between the jet nozzle of the top part and the tubular structure of the base part has a width of less than 2 mm, preferably less than about 1 mm.
- the inner shape of the jet nozzle of the top part matches the outer shape of the tubular structure of the base part, preferably they are (tronco)conical shapes.
- the spacing is delimited by the inner shape of the jet nozzle of the top part and the outer shape of the tubular structure of the base part, preferably the spacing has a conical shape.
- the tubular structure of the base part comprises an nozzle portion comprising a compressed-gas outlet.
- the jet nozzle of the top part is arranged as a sleeve around nozzle portion of the base part.
- the jet outlet of the jet nozzle of the top part and the compressed-gas outlet of the nozzle portion of the base part are coaxially-arranged (axis XX).
- the compressed-gas outlet of the nozzle portion of the base part provides compressed gas, typically compressed air.
- the jet outlet of the jet nozzle of the top part is arranged as so to face the compressed-gas outlet of the nozzle portion of the base part so that a compressed-gas flow delivered by the compressed-gas outlet of the nozzle of the base part passes through the jet outlet of the jet nozzle of the top part.
- the base part comprises a circular rim.
- the circular rim of the base part is located at the upper end of the base part.
- the top part comprises a collar structure projecting outwardly.
- the collar structure is arranged on the enlarged head of the top part.
- the collar structure of the top part comprises an annular lower shoulder coming to rest (i.e. abutting) on the circular rim of the base part, when the top part is attached to the base part.
- the circular rim of the base part comprises said at least one abutment wall of the base part, preferably the first and second abutment walls are arranged or make part of the circular rim of the base part.
- the annular lower shoulder of the collar structure of the top part is (axially-) spaced from said at least one protruding element by at least one spacing lodging, preferably each protruding element is spaced from the annular lower shoulder of the collar structure by a spacing lodging, namely a first and a second spacing lodging.
- the at least one, preferably each, abutment wall of the base part is lodged in said at least one, preferably each, spacing lodging and sandwiched between said at least one, preferably each, protruding element and the collar structure, when the top part is secured to the base part, namely when the top part is in the locking position, preferably.
- at least one axial groove is arranged in the inner wall of the base part, preferably a first and a second axial grooves
- said at least one axial groove, preferably each axial groove, is formed in the inner wall of the base part by (injection-)molding or similar.
- said at least one axial groove is associated to at least one spacing lodging.
- each axial groove is associated to a given spacing lodging.
- said at least one axial groove communicates with said at least one spacing lodging so that said at least one protruding element can be inserted in said at least one spacing lodging via said at least one axial groove.
- each axial groove communicates with a spacing lodging so that said each protruding element can be inserted in a given spacing lodging via a given axial groove.
- according to a preferred embodiment, the first and second protruding elements have different shapes and/or sizes.
- according to another embodiment, the first and second protruding elements have same shapes and/or sizes.
- each protruding element, in particular the first and second protruding elements, is (are) arranged on an outer peripheral surface of the top part, preferably arranged on the enlarged head of the top part.
- ach protruding element, in particular the first and second protruding elements are made one piece with the top part, in particular with the enlarged head of the top part, by (injection-) molding or similar.
- the first and second protruding elements are shaped and/or sized for allowing their motion in corresponding axial grooves and spacing lodgings, when the top part is attached to or detached from the base part by a user, namely when the user manipulates the top part, for instance turns it clockwise or counterclockwise.
- the tubular body of the base part comprises an inner volume comprising the liquid chamber.
- the liquid chamber forms a bottom part of the inner volume of the base part.
- the top part is inserted in said inner volume of the base part when the top part is coupled to the base part, i.e. assembled together.
- the first and second protruding elements are, comprise or constitute dents, picots, fingers, pins, bossings, short walls or similar.
- the first and second abutment wall are little walls or similar, preferably arcuate little wall.

According to other embodiments, the nebulizer according to the present invention can further comprise one or several of the following additional features:
- the liquid chamber (i.e. ampoule chamber) of the base part has an inner volume of between 30 cm³ and 60 cm³.
- the liquid chamber is configured to store at least 8 mL of liquid solution, preferably at least 10 mL.
- the jet nozzle of the aerosol-generating system comprises a jet orifice.
- the elongated tubular portion comprises a compressed-gas outlet having a diameter of between 0, 4 and 0,6 mm, preferably 0,45 mm and 0,54 mm.
- the tubular structure projects upwardly at the center of the liquid chamber of the base part, preferably axially-arranged along axis XX.
- the liquid chamber has a cylindrical or tonconical shape.
- the inner conduit projecting upwardly in the liquid chamber of the base part further comprises a compressed-gas inlet, such as a compressed air inlet.
- the inner conduit of the base part further comprises, i.e. is axially-traversed by, a compressed-gas passage (i.e. lumen) fluidly connecting the compressed-gas inlet and outlet, typically air inlet and outlet.
- the inner conduit of the base part is configured for being fluid connected to a compressed-gas delivery port of a gas-compressing device thereby providing compressed gas to the inner conduit of the base part, typically compressed-air.
- the aerosol-generating system of the top part further comprises a deflector arranged to face the jet orifice of the jet nozzle so that when the jet of compressed-air and liquid impacts the deflector, a mist, i.e. an aerosol, is created/generated.
- the top part, including the jet nozzle and the deflector, are made one-piece, for instance (injection-) molded in one piece.
- the nebulizer further comprises an aerosol chamber for recovering at least a part of the aerosol generated by the aerosol-generating system.
- the aerosol chamber is in fluid communication with a delivery conduit.
- the delivery conduit is in fluid communication with a respiratory interface for providing the aerosol to a user.
- the respiratory interface is a mouthpiece, or a nasal or facial mask.
- the jet nozzle comprises a lower rim located close to the bottom of the liquid chamber, i.e. at a very few distance, for instance less than 2 mm, preferably less than 1 mm so that the liquid, i.e. drug-containing solution, contained, i.e. stored, in said ampoule chamber can pass between said lower rim and said bottom and subsequently travels in the spacing between the jet nozzle and the tubular structure of the base part, namely between the inner wall of the jet nozzle and the outer wall of said tubular structure of the base part.
- the liquid chamber has a cup-like shape.
- the compressed-gas outlet of the elongated tubular structure is configured for accelerating the air flow thereby creating a Venturi or suction effect pulling some liquid or drug solution out of the liquid chamber.
- once pulled out of the liquid chamber by said Venturi or suction effect, the liquid, such as a liquid-drug, mixes with the compressed-air flow delivered by the tubular structure of the base part projecting upwardly and the resulting air/liquid jet is propelled onto the deflector, i.e. impactor, thereby creating the desired mist or aerosol.
- the mist contains droplets of liquid medication, i.e. liquid drug or dissolved drug particles.
- the elongated tubular portion of the top part comprises an inner channel comprising an upper outlet in fluid communication with the ambient atmosphere.
- the jet nozzle and the deflector of the aerosol-generating system and the elongated tubular portion of the top part are made one-piece, for instance molded in one-piece, preferably of polymer.
- the top part and the base part are at least partially made of polymer, such as polycarbonate (PC), polypropylene (PP), polyamide (PA), acrylonitrile butadiene styrene (ABS), polysulfone (PSU) or any other suitable plastic material.

The invention further concerns an aerosoltherapy installation useable for aerosolizing, i.e. nebulizing, a liquid, such as a drug-containing solution, comprising a nebulizer according to the present invention, and a gas-compressing device for providing a compressed gas to the nebulizer, preferably compressed air.

Depending on the embodiment, the gas-compressing device, i.e. also called compressor device, of the aerosoltherapy installation according to the present invention can comprise one or several of the following additional features:
- it is driven by an electric motor.
- the electric motor comprises a stator and a rotor.
- the stator and the rotor are arranged in a protective housing, such as a polymer casing or the like.
- it further comprises compressing elements driven by the electric motor for compressing the gas, typically ambient air.
- the compressing elements comprise a compressor member, such as a piston element or the like.
- the compressing elements further comprise a compression chamber.
- the piston element mobile is mobile in the compression chamber, preferably according to a back and forth motion, for sucking and compressing air.
- the compressor member, especially the piston element, is -directly or indirectly-fixed to the rotatable shaft of the electric motor.
- the compressing elements further comprise a manifold block for providing air to the compression chamber.
- the gas-compressing device is configured for compressing gas, preferably ambient air, to a pressure of beteween 1,2 and 3,5 bar abs.
- the electric motor is powered with electric power, for instance electric current of between 110 and 220 volt.
- it comprises a compressed-gas delivery port for providing compressed gas, e.g. compressed air, to the nebulizer, preferably to the inner passage or lumen of the central tubular structure of the base part that projects upwardly in the liquid chamber.
- the compressed-gas delivery port is fluidly connected to the nebulizer by means of a flexible hose or the like.

Embodiments of a nebulizer according to the present invention are shown in the enclosed Figures, among which:
- Figure 1 represents an embodiment of a nebulizer according to the present invention.
- Figure 2 represents a sectional view of the nebulizer of Fig. 1.
- Figure 3 shows an embodiment of the top part of the nebulizer of Fig. 1.
- Figure 4 is an internal view (from above) an embodiment of the base part of the nebulizer of Fig. 1.
- Figure 5 shows a partial lateral view of the base part of Fig. 4.
- Figure 6 represents an embodiment of a filter module shown separated from the nebulizer of Fig. 1, according to the present invention.
- Figure 7 shows the filter module of Fig. 6 partially coupled to the nebulizer of Fig. 1, according to the present invention.
- Figure 8 is a cross-sectional schematic view of the filter module of Fig. 6 & 7.

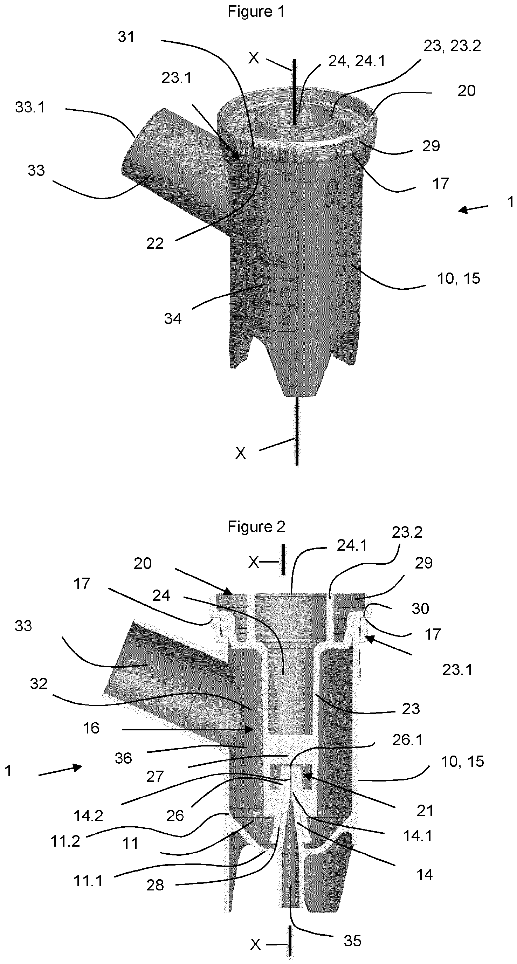

Figures 1 and 2 show an embodiment of a nebulizer 1 according to the present invention that can be fluidly connected to an air-compressing device (not shown), i.e. an air compressor, by a flexible hose (not shown) or the like that conveys the flow of compressed-air provided by the air-compressing device to the nebulizer 1 for generating a desired aerosol, as below explained.

In the nebulizer 1, the compressed-air flow is used for atomizing a liquid, typically a drug-containing solution to be aerosolized, that is subsequently delivered to a patient in need thereof, typically by means of a respiratory interface, such as a mouthpiece or a nasal or facial mask (not shown).

Typically, an air-compressing device comprises a rigid casing containing compressing elements driven by an electric motor that are used for compressing ambient air (i.e. air at atmospheric pressure) thereby delivering a flow of compressed air, typically at a pressure above atmospheric pressure. The electric motor, such as a brushless motor, is powered by a power source (not shown) providing electric current, such as the mains, i.e. 110/220V. Compressing elements can comprise an air compression chamber, a compressor element, such as a mobile piston element or the like, driven by the electric motor, and preferably a manifold block cooperating together for sucking and compressing ambient air. Ambient air sucked by the compressor element driven by the electric motor, passes through the manifold block, then enters into the air compression chamber where it is compressed by the compressor element and, once compressed, exits the air compression chamber and is conveyed to the nebulizer 1, via a flexible hose. Such an arrangement is known and described by EP4206467.

As shown in Fig. 1&2, the nebulizer 1 of the present invention, that is used for aerosolizing, i.e. nebulizing, a liquid, such as a drug-containing solution, is made of sub-units detachably attached together, namely, here, a top part 20 and a bottom part 10.

The top part 20 is at least partially inserted in the bottom or base part 10 and maintained therein. Both have generally-cylindrical shapes and are coaxially arranged on a common axis XX, i.e. a vertical axis XX.

The base part 10 comprises a tubular body 15, i.e. having a cylindrical shape, comprising an inner volume 16 including a liquid chamber 11 for storing a liquid to be aerosolized, i.e. a drug-containing solution, and a central tubular structure projecting upwardly in the liquid chamber 11. Preferably, the liquid chamber 11 forms a bottom part of the inner volume 16 of the base part 10.

Further, the top part 20 comprises an elongated tubular portion 23, i.e. having a cylindrical shape, and an aerosol-generating system 21.

The aerosol-generating system 21 comprises a deflector 27 and a jet nozzle 26 arranged as a sleeve around at least a part of the elongated tubular structure 14 of the base part 10, namely around the top end of the tubular structure 14 forming a nozzle portion 14.1 comprising a compressed-gas outlet 14.2 that provides a compressed gas, typically compressed air provided by the air compressor. The compressed-gas outlet 14.2 of the elongated tubular structure has a diameter of between 0,4 and 0,6 mm, preferably 0,45 mm and 0,54 mm.

The jet nozzle 26 delivers a pressurized jet of liquid/gas toward the deflector 27 so that said jet can impact the deflector 27.

The jet outlet 26.1 of the jet nozzle 26 of the top part 20 and the compressed-gas outlet 14.2 of the nozzle portion 14.1 of the base part 10 are coaxially-arranged (i.e. axis XX) and facing each other so that compressed-air delivered by the compressed-gas outlet 14.2 of the nozzle portion 14.1 of the base part 10 passes subsequently through the jet outlet 26.1 of the jet nozzle 26 of the top part 20, thereby sucking a part of the liquid out of the liquid reservoir 11 and creating the desired aerosol when impacting the deflector 27, as below explained.

The top part 20 is detachably secured to the base part 10 so that both parts 10, 20 can be detached, when desired, for refilling the liquid reservoir 11, for cleaning operations or for any other purposes. In this aim, the top part 20 and the base part 10 comprise an improved connecting system that avoids wrong attachments.

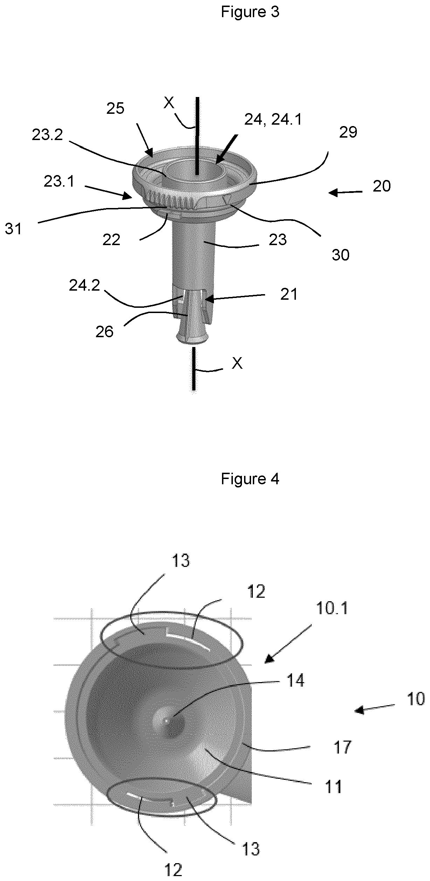

One the one hand, the top part 20 further comprises at least one protruding element 22 projecting radially and outwardly, namely away from axis XX as better shown in Fig. 3.

Preferably, in the present embodiment, two protruding elements 22, i.e. a first and a second protruding element 22, are arranged on the top part 20. They are diametrically-opposite arranged on the elongated tubular portion 23 of the top part 20.

The protruding elements 22 are located on the top end region 23.1 of the elongated tubular portion 23 of the top part 20. The protruding elements 22 can be little structures, such as dents, picots, fingers, pins, bossings, little walls or similar.

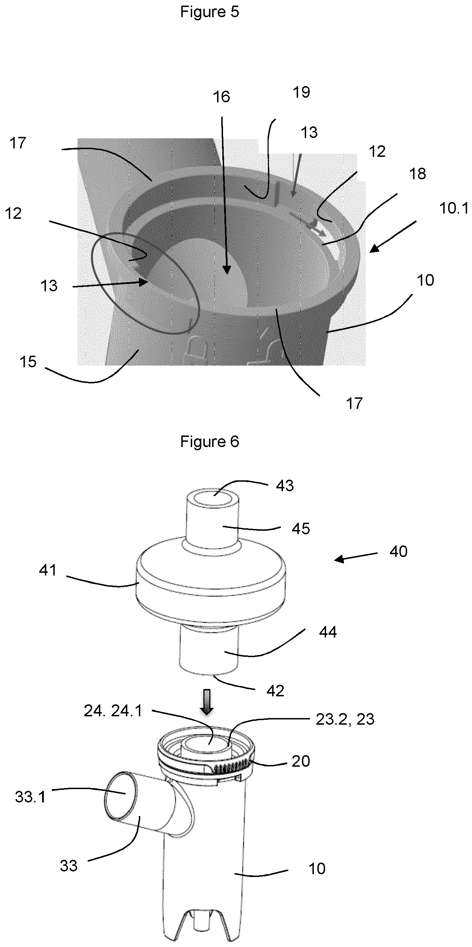

On the other hand, the base part 10 further comprises at least one abutment wall 12, such as little walls or the like, projecting radially and inwardly, namely toward axis XX as better shown in Fig. 5.

Preferably, in the present embodiment, two abutment walls 12, i.e. a first and a second abutment wall 12, are arranged on the base part 10. They are diametrically-opposite arranged in the tubular body 15 of the base part 10.

Actually, the two protruding elements 22, i.e. the first and second protruding elements 22, respectively, cooperate with the two abutment walls 12, i.e. the first and second abutment walls 12, respectively, for maintaining the top part 20 secured to the base part 10, as below explained.

Preferably, the top part 20 is mobile (at least) in rotation with respect to the base part 10 between at least a locking position and an unlocking position.

In the locking position, the top part 20 is firmly secured to the base part 10 thanks to the cooperation of the protruding elements 22 with the abutment walls 12 as the protruding elements 22 abut against the abutment walls 12 for preventing any detachment of the top part 20 from the base part 10.

In contrast, in the unlocking position, the top part 20 can be released and detached from the base part 10 as the protruding elements 22 do not abut anymore against the abutment walls 12.

As shown in Fig. 2&3, the top part 20 further comprises a collar structure 29 projecting outwardly, i.e. away from the axis XX, and comprising an annular lower shoulder 30 coming to rest, i.e. abutting, on a circular rim 17 of the base part 10, when the top part 20 is attached to the base part 10, in particular when in the locking position.

The circular rim 17 is located, as shown in Fig. 4&5, at the upper end 10.1 of the base part 10. The circular rim 17 comprises the abutment walls 12 of the base part 10. In other words, in the present embodiment, the abutment walls 12 make part of the circular rim 17.

When in the locking position, the abutment walls 12 are sandwiched between the collar structure 29 and the protruding elements 22, thereby the top part 20 and the base part 10 maintaining firmly secured together. Preferably, the collar structure 29 comprises an at least partially-circular outer periphery, i.e. contour.

More precisely, the annular lower shoulder 30 of the collar structure 29 of the top part 20 is axially-spaced (XX) from the protruding elements 22 by spacing lodgings 31. The abutment walls 12 of the base part 10 are lodged in said spacing lodgings 31 and sandwiched between the protruding elements 22 and the collar structure 19 when the top part 20 is secured to the base part 10, namely when in the locking position.

Further, as shown in Fig. 4&5, axial grooves 13, namely here a first and a second axial groove 13, are arranged in the inner wall 19 of the base part 10, preferably approximatively the axial grooves 13 are axially-oriented (XX).

Each axial groove 13 is associated to a spacing lodging 31 and communicates with said corresponding spacing lodging 31 so that the protruding elements 22 can be inserted in said spacing lodgings 31 via said axial grooves 13.

In other words, for locking the top art 20 to the base part 10, a user has first to insert the protruding elements 22 in corresponding axial grooves 13 that constitutes guides or the like, preferably upon an axial motion in translation. This can be obtained in grabbing the top part 20, in particular its enlarged head 25 as below explained, in one hand and the bottom part 10 in the other hand.

Then, the user has to operate a rotational motion, i.e. to turn the top part 20, especially its enlarged head 25, for instance clockwise, so that the protruding elements 22 can travel and enter into said spacing lodgings 31, until the locking position is reached.

The first and second protruding elements 22 have preferably different shapes and/or sizes, and said axial grooves 13 have corresponding shapes, for constituting a poka-yoke system or the like avoiding bad connections, i.e. an anti-error system.

Anyway, the first and second protruding elements 22 are shaped and/or sized for allowing their motion/travel in corresponding axial grooves 13 and spacing lodgings 31, when the top part 20 and base part 10 are manipulated by the user, i.e. when the top part 20 is attached to or detached from the base part 10 by a user, in particular when passing from the unlocking position to the locking one, or vice versa.

As shown in Fig. 3, the top part 20 comprises an enlarged head 25 that has, in the present embodiment, a disk-shape. The enlarged head 25 carries the protruding elements 22 and further comprises the collar structure 29.

Further, the top part 20 is traversed by an inner channel 24 comprising an upper outlet 24.1 that is arranged in said enlarged head 25. Preferably, the inner channel 24 is axially-arranged (axis XX) in the elongated tubular portion 23 of the top part 20.

The deflector 27 of the aerosol-generating system 21 is arranged in the region of the entrance 24.2 of the inner channel 24 arranged in the elongated tubular portion 23 as shown in Fig. 2 and 3. The deflector 27 and the jet outlet 26.1 of the jet nozzle 26 are arranged face to face.

Advantageously, at least the deflector 27, the jet nozzle 26 and the elongated tubular portion 23 of the top part 20 are made one piece, preferably the top part 20 is entirely made in one piece, for instance (injection-)molded in one piece.

As already explained, the jet nozzle 26 of the top part 20 is arranged as a sleeve around the tubular structure 14 of the base part 10, namely around the nozzle portion 14.1 comprising the compressed-gas outlet 14.2, but at a distance from each other. Indeed, a spacing 28 is maintained in-between so that liquid stored in the liquid chamber 11 can travel upwardly in said spacing 28 toward the jet outlet 26.1 of the jet nozzle 26, when it is sucked out of the liquid chamber 11 by the gas sucking-pressure.

As shown in Fig. 2, the liquid chamber 11 is delimited by peripheral 11.2 and bottom 11.1 walls forming a reservoir or tank that receives and stores the liquid L to be aerosolized. Preferably, the ampoule or liquid chamber 11 has a "cup-like" shape traversed by the tubular structure 14, such as an inner conduit or tube, carrying the nozzle portion 14.1 and the compressed-gas outlet 14.2, that is preferably arranged at the center of the liquid chamber 11 and projects axially (XX) and upwardly into the liquid chamber 11 of the nebulizer 10.

Advantageously, the base part 10 forming the lower part of the nebulizer 1 is made in one piece, for instance (injection)molded in one piece. However, according to another embodiment, the liquid chamber 11 can be detachable, for instance it can constitute a removable reservoir that is detachably attached to the bottom part of the inner volume 16 of the base part 10 of the nebulizer 1.

Generally speaking, the liquid chamber 11 can containing a given amount of liquid to be aerosolized, typically a drug-containing solution or the like, for instance less tan 20 mL, preferably 10 mL or more. The amount of liquid present in the liquid chamber 11 can be visualized by means of graduations or markings 34 arranged on the outer surface of the base part 10, for instance from 0 to 10 ml, as shown in Fig. 1.

The tubular structure 14 has a tubular shape, i.e. outer shape, preferably a cylindrical, conical or tronconical shape.

Further, the tubular structure 14, i.e. inner conduit or tube, traversing the liquid chamber 11 comprises an inner passage or lumen 35 in fluid communication with the air compressor, that conveys compressed air to the compressed-gas outlet 14.2 for creating the desired mist or aerosol, downstream of said compressed-gas outlet 14.2, thanks to the aerosol-generating system 21, with liquid pulled out of the liquid chamber 11 and said compressed air.

As already mentioned, the aerosol-generating system 21 comprises the jet nozzle 26 with the jet outlet 26.1, i.e. a delivery orifice for delivering a jet of liquid/compressed air toward the deflector 27, also called impactor or pisper, arranged to face the jet outlet 26.1 of the jet nozzle 26. The deflector 27, can have various shapes, for instance it can be or comprise a little wall, a blade, a pin or any other suitable shape.

Further, the jet outlet 26.1 can have a circular section, an ellipse section or any other suitable shape, preferably a circular section.

As the jet nozzle 26 is arranged as a sleeve around the nozzle portion 14.1 of the tubular structure 14, but at a distance of less than 2 mm, preferably a width of less than about 1 mm, a little spacing 28 is kept between the inner wall or surface of the jet nozzle 26 and the outer wall or surface of the tubular structure 14, some liquid stored in the liquid chamber 11 can travel in said little spacing 28, when said liquid is sucked out by Venturi effect as below explained. The little spacing 28 has an annular section or the like, and/or a conical shape or the like.

Further, the lower end of the jet nozzle 26 projects into the liquid chamber 11 containing the liquid drug and terminates close to the bottom wall 11.1 of said liquid chamber 11, as shown in Figure 2, preferably at a distance of less than about 2 mm, thereby allowing the liquid to circulate between the surface of the bottom wall 11.1 and the rim forming the lower end of the jet nozzle 26. Thus, the liquid contained into the liquid chamber 11 is able to circulate, i.e. to pass, in-between and then enter and further travel into the little spacing 28, before reaching the aerosol-generating system 21, in particular before impacting the deflector 27 that is configured for generating the aerosol or mist.

The principle of the Venturi effect that takes place in the nebulizer 1 is well-known in the art. In few words, as the compressed air jet is delivered at a high speed and at a constant flowrate by the compressed-gas outlet of the nozzle portion 14.1, a suction effect is generated that moves, i.e. pulls out, liquid solution out of the liquid chamber 111. The liquid travels in the little spacing 28 as it is upwardly pulled by the suction effect and is then delivered as a jet of liquid/air by the jet outlet 26.1 of the jet nozzle 26 toward the deflector 27, such as a blade or similar, thereby creating the desired mist, i.e. aerosol, that is formed of droplets of liquid, i.e. drug or the like, carried by the air stream.

Indeed, when the jet of liquid/air enters into contact with the deflector 27, it "explodes" thereby forming a mist of liquid droplets is created by the impact of the jet of liquid/air on the deflector 27.

The compressed-gas outlet 14.1 of the nozzle portion 14.1 of the elongated tubular portion 14 comprises has preferably a precise diameter for ensuring a suitable particle size of the droplets forming the mist as well as an efficient drug amount in the mist. For instance, it has an orifice diameter of less than 0.55 mm, preferably of between 0.45 mm and 0.54 mm.

The obtained mist or aerosol continues its journey toward the patient's airways, via an aerosol chamber 36 on Fig. 2, for instance the aerosol chamber 36 makes part of the inner volume 16 of the nebulizer 1.

The aerosol chamber 36 recovering the aerosol mist is in fluid communication with a delivery conduit 33 fluidly connected to a mouthpiece (not shown) for providing the aerosol to the airways of a user, i.e. a patient inhaling the aerosol. The mouthpiece is in fluid communication with the outlet 33.1 of the delivery conduit 33.

However, for increasing the volume of gas inhaled by the user, i.e. the patient, the mist or aerosol thus created is preferably mixed with additional air provided by the elongated tubular portion 23 of the top part 20.

Indeed, when the patient inhales through the delivery conduit 33 connected to a mouthpiece or the like, a depression appears in the delivery conduit 33 and in the inner channel 24 of the elongated tubular portion 23 which are in fluid communication. Then, ambient air is sucked in the nebulizer 1 and a flow of additional air enters into the inner channel 24 of the elongated tubular portion 23 of the top part 20, through the upper outlet 24.1 arranged in the enlarged head 25 and in fluid communication with the ambient atmosphere.

Said flow of additional air, mixes with the mist created in the region of the deflector 27 and the resulting aerosol travels to the airways of the patient, via the aerosol chamber 36 and the delivery conduit 33

The aerosol can thereafter be inhaled by the patient thanks to a respiratory interface that can be a mouthpiece, or a mouth, nasal or facial respiratory mask that can be attached to the delivery conduit 33. The choice of the most suitable interface depends on the type of medication to be taken and on the type of patients, for instance a facial mask is advocated for pediatric patients, for instance children or toddlers, while a mouthpiece or a mouth mask is more suitable for adults.

Generally speaking, the different components of the nebulizer 1, in particular the top part 20 and the base part 10, are preferably made of plastic material(s), such as PP, PC, ABS, PA, PSU or similar materials.

The nebulizer 1 of the present invention is usable for nebulizing a liquid drug- or medication-containing solution, thereby obtaining a mist or aerosol containing droplets of medication having suitable sizes so as to be able to reach the lower airways of the patient(s) that has/have to inhale said mist in the course of their aerosoltherapy.

During expiratory phases, when the patient exhales, i.e. expires, CO₂-containing gases through the delivery conduit 33 connected to a mouthpiece or the like. The flow of expired gases travels successively in the delivery conduit 33 and in the inner channel 24 of the elongated tubular portion 23 which are in fluid communication, before being released to the ambient atmosphere through the upper outlet 24.1 located at the end of the inner channel 24 of the elongated tubular portion 23 of the top part 20.

However, those CO₂-enriched expired gases generally also contain water vapor and some remaining medication(s), i.e. drug compounds or the like, that have not been retained by the patient's airways, such as the lungs. Once vented to the atmosphere during each expiratory phase of the patient, said medications tended to accumulate in the air of the room where the patient and/or other people are, such as family members, medical staff or others, which can be a problem as they may be inhaled by those people, especially by people who don't need them.

In order to avoid or to limit that all the medications/drug compounds present in expired gases (i.e. besides CO₂ and water vapor) are released to the atmosphere, according to the present invention, a filter module 40 is detachably secured to the top part 20 of the nebulizer 1, as shown in Fig. 6-8.

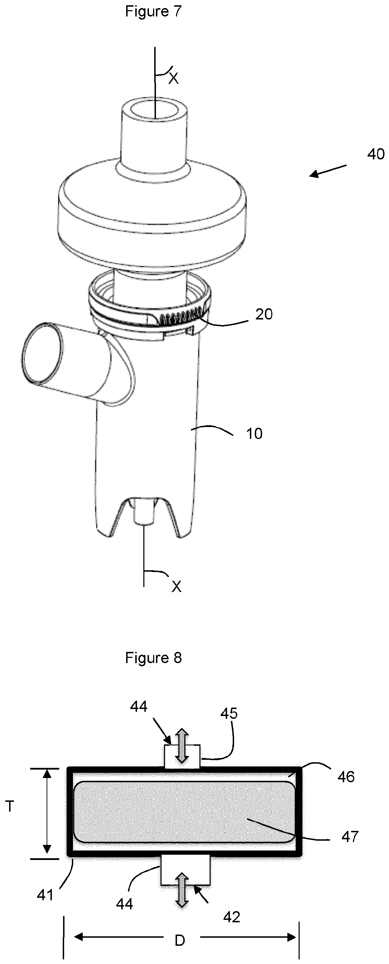

The filter module 40 comprises a module body 41 defining an inner chamber 46 (Fig. 8), i.e. a hollow volume, containing a filter material 47.

The module body 41 further comprises a first and a second opening 42, 43 that are in fluid communication with the inner chamber 46 thereby allowing the flow of expired gases to successively pass through the first opening 42, enter the inner chamber 46 of the module body 41, pass through the filter material and is vented to the atmosphere via the second opening 43.

The first and second openings 42, 43 are located on a first and a second tubular connector 44, 45, i.e. hollow little tubes, cylinders or the like, attached to the module body 41 of the filter module 40.

As shown in Fig. 6, the first tubular connector 44 of the module body 41 can be secured to, e.g. plugged onto (as a sleeve), the tubular top end 23.2 of the top end region 23.1 of the elongated tubular portion 23 of the top part 20 of the nebulizer 1, for instance, by a press-fit connection or the like.

In this aim, the inner wall of the first tubular connector 44 of the module body 41 comprises an inner shape or profile that is complementary to the outer shape or profile of the tubular top end 23.2 of the top end region 23.1 of the elongated tubular portion 23 of the top part 20 of the nebulizer 1 so that they can be plugged one into the other, i.e. interlocked or the like, i.e. their respective surfaces mate with one another (i.e. matching surfaces).

Typically, the inner shape or profile of the first tubular connector 44 and the outer shape or profile of the tubular top end 23.2 of the elongated tubular portion 23 of the top part 20 are cylindrical or similar.

Preferably, the inner diameter of the first tubular connector 44 and the outer diameter of the tubular top end 23.2 of the elongated tubular portion 23 of the top part 20 are approximatively equal so that the elongated tubular portion 23 can be axially (axis XX) interted and secured into the first tubular connector 44. Fig. 7 illustrates the filter module 40 attached to the top part 20 of the nebulizer 1.

Further, the inner chamber 46 contains a filter material 47 that can be made of fibers or the like, typically polymer fibers, such as styrene butadiene copolymer (SBC) or polypropylene.

Furthermore, the module body 41 can be made of a plastic material or the like, such as PC, PP or PA.

In the embodiment of Fig. 6-8, the module body has a cylindrical shape with a diameter D of between about 2 and 8 cm, and a thickness T of between about 2 and 10 cm.

The module body 41 can be further made of several sub-parts that are secured together, for instance glued together, in particular for allowing the insertion of the filter material 47 into the inner chamber 46 of the module body 41.

While expired gases pass through the module body 41, during the expiration phases of the user, the drug compounds present in expired gases can be trapped/retained, i.e. filtered, by the filter material in lieu of being vented to the atmosphere.

As the second tubular connector 45 of the filter module 40 is in fluid communication with the ambient atmosphere, the expired-gases (i.e. CO₂ enriched gases) that are substantially free of drug compounds, can be subsequently vented, i.e. released, to the atmosphere.

The filter module 40 can be detached from the filter module 40 after a given period of time and replaced by a new one, for instance after saturation of the filter material 47 and/or after each session of use of the nebulizer 1 by the user.

Generally speaking, the nebulizer 1 according to the present invention is designed for being hold in hand by a user and is useable in aerosoltherapy for aerosolizing or nebulizing a liquid, such as a drug- or medication-containing liquid or solution, when driven by the air-compressing device of an aerosoltherapy installation.

## Claims

1. Nebulizer (1) for aerosolizing a liquid comprising :
- a base part (10) comprising a tubular body (15) comprising a liquid chamber (11) for storing a liquid to be aerosolized and a central tubular structure (14) projecting upwardly in the liquid chamber (11), and
- a top part (20) comprising an elongated tubular portion (23) comprising an inner channel (24),
and an aerosol-generating system (21), said aerosol-generating system (21) comprising a jet nozzle (26) arranged as a sleeve around at least a part of the tubular structure (14) of the base part (10), and
wherein the top part (20) is detachably secured to the base part (10),
**characterized in that** it further comprises a filter module (40) detachably attached to the top part (20), wherein:
- the filter module (40) comprises a module body (41) comprising an inner chamber (46) comprising a filter material (47) and
- the inner chamber (46) of the module body (41) is in fluid communication with the inner channel (24) of the elongated tubular portion (23).

2. Nebulizer according to Claim 1, **characterized in that** the inner channel (24) comprises an upper outlet (24.1) arranged in a tubular top end (23.2) of the elongated tubular portion (23).

3. Nebulizer according to Claim 1, **characterized in that** the module body (41) of the filter module (40) comprises a first and a second opening (42, 43) in fluid communication with the inner chamber (46).

4. Nebulizer according to Claim 3, **characterized in that** the first opening (42) is arranged on a first tubular connector (44) and/or the second opening (43) is arranged on a second tubular connector (45), said first and second tubular connectors (44, 45) being integral with the module body (41) of the filter module (40).

5. Nebulizer according to Claim 1 and 4, **characterized in that**, when the filter module (40) is attached to the top part (20), the first tubular connector (44) of the module body (41) is in fluid communication with the inner channel (24) of the elongated tubular portion (23) and with the inner chamber (46) of the module body (41).

6. Nebulizer according to Claim 2 and 4 or 5, **characterized in that** the first tubular connector (44) of the module body (41) is plugged onto the tubular top end (23.2) of the elongated tubular portion (23), when the filter module (40) is attached to the top part (20).

7. Nebulizer according to Claim 1, **characterized in that** the top part (20) is mobile in rotation with respect to the base part (10) between at least: a locking position, wherein the top part (20) is secured to the base part (10), and an unlocking position, wherein the top part (20) is released and can be detached from the base part (10).

8. Nebulizer according to Claim 2, **characterized in that** the top part (20) comprises an enlarged head (25) comprising the tubular top end (23.2) of the elongated tubular portion (23).

9. Nebulizer according to Claim 1, **characterized in that** the filter material (47) comprises polymer fibers.

10. Nebulizer according to Claim 9, **characterized in that** the filter material (47) comprises polymer fibers of styrene butadiene copolymer (SBC) or polypropylene.

11. Nebulizer according to Claim 4, **characterized in that** the second opening (43) of the second tubular connector (45) is in fluid communication with the ambient atmosphere.

12. Nebulizer according to Claim 1, **characterized in that** the aerosol-generating system (21) of the top part (20) further comprises a deflector (27) facing a jet outlet (26.1) of the jet nozzle (26).

13. Nebulizer according to Claim 1, **characterized in that** the jet nozzle (26) of the top part (20) is arranged as a sleeve around the tubular structure (14) of the base part (10), while maintaining a spacing (28) in-between so that liquid stored in the liquid chamber (11) can travel upwardly in said spacing (28) toward the jet outlet (26.1) of the jet nozzle (26).

14. Nebulizer according to Claim 1, **characterized in that** the tubular body (15) of the base part (10) further comprises an aerosol chamber (36) for recovering at least a part of the mist created by the aerosol-generating system (21) of the top part (20), said aerosol chamber (36) being a in fluid communication with a delivery conduit (33) for providing the aerosol to the user.

15. Aerosoltherapy installation useable for aerosolizing a liquid, such as a drug-containing solution, comprising a nebulizer (1) according to any one of the previous Claims, and a gas-compressing device for providing a compressed gas to the nebulizer (1), preferably compressed air.
